# EUROPEAN PATENT SPECIFICATION

(11) **EP 0 349 276 B1**
(45) Date of publication and mention of the grant of the patent: **22.03.1995**
(21) Application number: 89306527.6
(22) Date of filing: 27.06.1989
(51) Int. Cl.: C07C 2/08, C07C 2/22

(54) **Olefin oligomer synlube process**
Verfahren zur Herstellung eines künstlichen Schmierstoffes aus Olefinoligomeren
Procédé pour la fabrication d'un lubrifiant synthétique d'oléfines oligomères

(30) Priority: 27.06.1988 US 212020
(43) Date of publication of application: 03.01.1990
(73) Proprietor: ALBEMARLE CORPORATION, Richmond, Virginia 23219 (US)
(72) Inventor: Lynch, Matthew Joseph, Baton Rouge Louisiana 70809 (US); Dileo, Thomas Joseph, Baton Rouge Louisiana 70814 (US); Lin, Ronny Wen-Long, Baton Rouge Louisiana 70817 (US); Nelson, Marshall Budd, Baton Rouge Louisiana 70816 (US)
(74) Representative: Woods, Geoffrey Corlett

(56) References cited:
- WO-A-82/04040
- US-A- 4 045 507
- US-A- 4 045 508

## Description

Alpha-olefin oligomers and their use as synthetic lubricants ("synlubes") are well-known. Early reports of such synlubes are in Seger et al. U.S. 2,500,161 and Garwood U.S. 2,500,163. U.S. 2,766,312 describes the oligomerization of α-olefins in a Group IV metal oxide bed using a BF₃ promoter catalyst. Promoters include water, alcohol and ether.

U.S. 3,149,178 describes the preparation of a synlube by oligomerizing a C₆₋₁₆ α-olefin thermally or using a Friedel Crafts or peroxide catalyst followed by distillation to remove dimer. The distillation residue is hydrogenated for use as a synlube. U.S. 3,382,291 discloses a BF₃-promoter (e.g., alcohol) process for making α-olefin oligomers in which the BF₃ is used to saturate the α-olefin feed and a second stream of BF₃-promoter is fed to the reaction.

Cupples U.S. 4,045,507 and U.S. 4,045,508 describe a continuous process of oligomerizing α-olefins using a BF₃-alcohol system.

WO82/04040 describes a process for oligomerizing an α-olefin having 3 to 12 carbon atoms, e.g. 1-decene, by contacting the α-olefin with a three component catalyst comprising boron trifluoride, a solid adsorbent and water.

Conventional processes of oligomerizing 1-decene generally give an oligomer which, after removal of monomer and dimer and hydrogenation, has a viscosity of about 5 centistokes (cs) at 100°C. This is somewhat high to qualify as a low viscosity synlube (appx. 4 cs required) and not high enough to qualify as a medium viscosity synlube (appx. 6 cs required). A useful way to make 6 cs synlube from the 5 cs product is to distill out a portion of the trimer until the required viscosity increase is obtained. This mode of operation required that some use be made of the trimer distillate.

According to the present invention a 6 centistoke hydrogenated 1-decene oligomer synlube is made by a process comprising:
(A) feeding 0.5-5 parts by weight 1-decene to 1 part by weight 1-decene trimer at a temperature of 20-35°C at a feed rate so that the feed period is at least 0.5 hour while in contact with a catalytic amount of BF₃ and a water or alcohol promoter,
(B) removing the BF₃ catalyst from the reaction mixture,
(C) distilling the reaction mixture to remove monomer and dimer, and
(D) hydrogenating the residual product to obtain a synlube having a viscosity at 100°C of 5.5-6.7 centistokes.

The trimer required to conduct the present process can be obtained from any source. A preferred source is a conventional α-olefin oligomer process in which 1-decene is oligomerized in the presence of a BF₃-promoter catalyst system. This will give a crude unsaturated oligomer consisting of 0-2 weight percent monomer, 1-5 weight percent dimer, 40-60 weight percent trimer, 20-35 weight percent tetramer, 10-15 weight percent pentamer and the balance is higher oligomers. the monomer and dimer can be removed and then the desired amount of trimer distilled out. Optionally the small amount of monomer and dimer can be included in the mainly trimer cut. Hereafter the trimer will be referred to merely as "trimer" but it is understood that it can contain minor amounts of monomer and dimer, up to ∼10 weight percent.

Many compounds are known to function as promoters for BF₃ in the α-olefin oligomerization process. These include water, alcohols (e.g., isopropanol, n-butanol, and decanol) polyhydric alcohol (e.g., glycol, glycerol, and trimethylolpropane), organic esters (e.g., methyl butyate, ethyl acetate, and methyl laurate), carboxylic acids (e.g., acetic acid, and valeric acid), alkoxylated alcohols (e.g., 2-methoxyethanol, 2-ethoxyethanol, and polyethoxylated (8) decanol), ethers (e.g., methyl isobutyl ether, and dioxane), ketones (e.g., methyl ethyl ketone), phosphorus acids (e.g., phosphoric acid), silica gel.

The promoters used in this invention are water and alcohol (e.g., n-butanol).

The amount of promoter is a promoter amount. A useful range is 0.1-2 weight percent based on the 1-decene and trimer used in the process. The promoter can be placed in the reactor with the 1-decene trimer feed, in the 1-decene feed, or in both the trimer and the 1-decene feed.

The reaction can be conducted by placing the trimer in a reaction vessel together with BF₃ and promoter and stirring at reaction temperature while feeding monomer. The useful temperature range is 20-35°C and preferably 25-30°C.

The weight ratio of monomer to trimer can vary from 0.5-5 parts monomer per each part trimer. A preferred weight ratio is 1-2 parts monomer per part trimer.

In a preferred mode, the BF₃ is provided by merely placing the reaction under BF₃ pressure of 70-140 kPa (10-20 psig).

The feed of 1-decene monomer to the trimer may be continuous or intermittent. The feed rate is controlled so that the feed period is at least 0.5 hour, more preferably at least one hour. This prevents the accumulation of monomer in the reaction mixture lessening the chances of monomer reacting with itself to form dimer.

The following example shows how the process can be conducted and the properties of the products. All parts are by weight.

### Example 1

This example shows the conventional preparation of an α-olefin oligomer. It is not the present process.

In an autoclave was placed 2000 parts of 1-decene and 14 parts of n-butanol. The autoclave was sealed and placed under 20 psig BF₃ pressure. Stirring was continued 2 hours at 30°C. The reaction product was water washed and contained:

| | weight percent |
|---|---|
| monomer | 1 |
| dimer | 2 |
| trimer | 50 |
| tetramer | 29 |
| pentamer | 13 |
| hexamer | 5 |

Monomer and dimer were distilled out and then 10 weight percent of the trimer was distilled as a starting material for Example 2.

### Examples 2-4

These examples embody the present invention.

In an autoclave was placed 500 parts of 1-decene trimer and varying amounts of n-butanol from 0.7 to 1.0 weight percent (refer to Table I). The autoclave was placed under 20 psig BF₃ pressure and stirred at 30°C while 750 parts of 1-decene, also containing varying amounts of n-butanol from 0.7 to 1.0 weight percent (refer to Table I), was fed to the autoclave at a constant rate over time periods from 3 to 6.5 hours (refer to Table I). Stirring was continued for 15 minutes after the 1-decene feed was complete. The autoclave was then vented and the oligomer product washed with aqueous caustic to remove BF₃ and butanol. It was then washed twice with water. The crude oligomer analyzed by gas chromatograph as shown in Table II.

**Table I**

| Example | n-butanol (wt %) in trimer and 1-decene | 1-decene feed time (hrs) |
|---|---|---|
| 2 | 0.7 | 3.0 |
| 3 | 1.0 | 6.25 |
| 4 | 1.0 | 3.0 |

**Table II**

| Example | Oligomer Composition (wt %) | | | | | |
|---|---|---|---|---|---|---|
| | C₁₀ | C₂₀ | C₃₀ | C₄₀ | C₅₀ | C₆₀ |
| 2 | 1.1 | 1.6 | 27.8 | 50.5 | 16.2 | 2.3 |
| 3 | 0.7 | 1.7 | 18.9 | 54.2 | 20.9 | 3.3 |
| 4 | 0.6 | 1.8 | 30.2 | 50.5 | 14.8 | 1.7 |

The crude oligomer was topped to remove monomer and dimer and then hydrogenated at 200°C under 400 psig using a nickel catalyst. The final composition and viscosities are shown in Table III.

**Table III**

| Example | Composition (wt %) | | | | Viscosity (cs) | |
|---|---|---|---|---|---|---|
| | C₃₀ | C₄₀ | C₅₀ | C₆₀ | 100°C | -40°C |
| 2 | 26.7 | 53.2 | 16.9 | 2.4 | 5.8 | 7760 |
| 3 | 18.6 | 55.5 | 21.5 | 3.6 | 6.2 | 9810 |
| 4 | 29.6 | 51.9 | 15.9 | 2.2 | 5.7 | 7550 |

These products meet the viscosity specifications for a 6 cs synlube.

## Claims

1. A process for making a 6 centistoke hydrogenated 1-decene oligomer synlube, said process comprising:
(A) feeding 0.5-5 parts by weight 1-decene to 1 part by weight 1-decene trimer at a temperature of 20-35°C at a feed rate so that the feed period is at least 0.5 hour while in contact with a catalytic amount of BF₃ and a water or alcohol promoter,
(B) removing the BF₃ catalyst from the reaction mixture,
(C) distilling the reaction mixture to remove monomer and dimer, and
(D) hydrogenating the residual product to obtain a synlube having a viscosity at 100°C of 5.5-6.7 centistokes.

2. A process according to claim 1 wherein said feed period is at least one hour.

## Patentansprüche

1. Verfahren zur Herstellung eines synthetischen Schmiermittels aus einem hydriertem 1-Decenoligomer mit einer Viskosität von 6 Centistokes, bei dem man
A) bei einer Temperatur von 20 - 35 °C 0,5 - 5 Gewichtsteile 1-Decen zu einem Gewichtsteil 1-Decentrimer mit einer solchen Zugabegeschwindigkeit, daß die Zugabe mindestens 0,5 Stunden dauert, bei gleichzeitigem Kontakt mit einer katalytischen Menge an BF₃ und einem Aktivator in Form von Wasser oder Alkohol zuführt,
B) den BF₃-Katalysator aus dem Reaktionsgemisch entfernt,
C) das Reaktionsgemisch zur Entfernung von Monomeren und Dimeren destilliert und
D) das verbliebene Produkt hydriert, um ein synthetisches Schmiermittel mit einer Viskosität von 5,5 - 6,7 Centistokes bei 100 °C zu erhalten.

2. Verfahren gemäß Anspruch 1, bei dem die Zugabe über eine Zeitspanne von mindestens einer Stunde erfolgt.

## Revendications

1. Procédé de préparation d'un lubrifiant de synthèse constitué d'un oligomère de 1-décène hydrogéné, ayant une viscosité de l'ordre de 6 centistokes, ledit procédé comprenant :
(A) la mise en contact de 0,5 à 5 parties en poids de 1-décène avec 1 partie en poids d'un trimère de 1-décène à une température de 20 à 35°C à une vitesse d'alimentation choisie de telle sorte que le temps d'alimentation soit au moins égal à 0,5 heure, en contact simultané avec une quantité catalytique de BF₃ et d'un activateur consistant en eau ou un alcool,
(B) la séparation du catalyseur consistant en BF₃ du mélange réactionnel,
(C) la distillation du mélange réactionnel pour éliminer le monomère et le dimère, et
(D) l'hydrogénation du produit résiduel pour obtenir un lubrifiant de synthèse ayant une viscosité, à 100°C, de 5,5 à 6,7 centistokes.

2. Procédé suivant la revendication 1, dans lequel le temps d'alimentation est au moins égal à une heure.
